# EUROPEAN PATENT APPLICATION

(11) **EP 2 083 008 A1**
(43) Date of publication of application: **29.07.2009**
(21) Application number: 09159841.7
(22) Date of filing: 07.12.2007
(51) Int. Cl.: C07D 457/06, A61K 31/48, C07D 457/04

(54) **Ergoline derivatives as selective radical scavengers for neurons**

(62) Divisional of application: 07122627.8
(71) Applicant: Axxonis Pharma AG, 10963 Berlin (DE)
(72) Inventor: Flieger, Miroslav, 1700, Praha 7 (CZ); Kranda, Karel, 14057, Berlin (DE); Latté, Klaus Peter, 13595, Berlin (DE); Pertz, Heinz, 10587, Berlin (DE); Horowski, Reinhard, 13353, Berlin (DE)
(74) Representative: Wablat, Wolfgang

(57) **Abstract**

The present invention relates to new Ergot alkaloid derivatives of the general formula (I) or pharmaceutically acceptable salts thereof as well as to the use of specific compounds as selective radical scavengers for neurons.

## Description

### Background of the invention

Neurodegenerative processes, such as Parkinson's disease (PD), Alzheimer's disease (AD), Huntington's disease (HD), amyotropic lateral sclerosis (ALS) but also ataxias and other chronic neurological disorders are characterised by a gradual and selective loss of particular classes of neurons that is progressing with age. These diseases are very common and their incidence also increases with age. The principal cause for this observation is that all these disease share some crucial steps in the pathogenetic cascade related to free radical damage.

As the aetiology of these neurological conditions is even to date poorly understood, the available options for a therapeutic intervention are at present largely limited to symptomatic treatment with various degrees of success. The effectiveness of current symptomatic treatments ranges from being relatively high, as in the case of PD, to very low as in the case of AD and nil as in HD.

As the principle causative factor of PD still remains to be discovered, then apart from a symptomatic treatment with L-dopa or dopaminergic agonists, the only other available strategy is to learn more about those mechanisms that are responsible for disease progression so that we may attempt to interfere with their function. Such an intervention, as we hope, can slow down the progression of not just PD but also the progression of other neurodegenerative diseases that in their more advanced stages, share with PD the common characteristics of a gradual but massive loss of dopaminergic and quite often other monoaminergic neurons.

Whatever the cause and course of the classical neurodegenerative diseases might indeed be, it is the common denominator of the progressive neuronal loss that may manifest an age-dependant decline of neuroprotective functions. Neuroprotection, as such is normally provided by the expression of endogenous mechanisms, such as enhanced proteasome activity, mitochondrial functions that supply NADH and energy, radical scavenging or anti-oxidant activity of some endogenous compounds. A malfunction of neuroprotective endogenous mechanisms is documented, by an early and rapidly progressing decline of the potent endogenous anti-oxidant glutathione as observed in substantia nigra during the early stages of dopaminergic cells loss in of PD.

Living organisms possess numerous protective mechanisms of that continually mop up free radicals and oxidants present in brain and other organs. This protective action may proceed either via enzymatic pathways utilising either the various superoxide dismutases (SOD1 to SOD3) or glutathione peroxidase (GSH-Px). Other processes may also utilise the oxidation of endogenous or exogenous molecules such as glutathione, ubiquinone vitamine E or ascorbic acid (Krinsky, N I (1992) Proc. Soc. Exp. Biol. Med. 200: 248-54).

Hence, apart from symptomatic treatment of PD, the most promising therapeutic strategy that could arrest or at least slow down neurodegenerative processes is to enhance the oxyradical (ROS) scavenging capacity of the brain by introducing compounds that can penetrate blood brain barrier and selectively reduce the level of superoxides in those brain structures that are vulnerable to exogenous toxins or susceptible to genetically induced disorders or to the combination of both factors. Although the action of numerous compounds has already been extensively examined for their presumed ability to slow down the progression neurodegeneration in affected patients, any apparent clinical success has so far been difficult to demonstrate in a convincing manner (for review see Suchowersky et al. 2006). To mention just few, glutathione, riluzol, melatonin, vitamine E, co-enzyme Q 10, cabergoline, pramipexole or the MAO-inhibitors selegiline and rasagiline have already been tested in numerous clinical trials to asses their neuroprotective potential in PD. Even though some of the above listed compounds are already being prescribed to patients to treat symptomatic effects, there is no generally accepted evidence of a neuroprotective action for these compounds or for any therapeutic strategy that is in use at present.

Thus, it is one object of the present invention to provide new compounds which have an improved neuroprotecive action.

The problem was solved by ergot alkaloid derivatives of the general formula (I) or pharmaceutically acceptable salts thereof 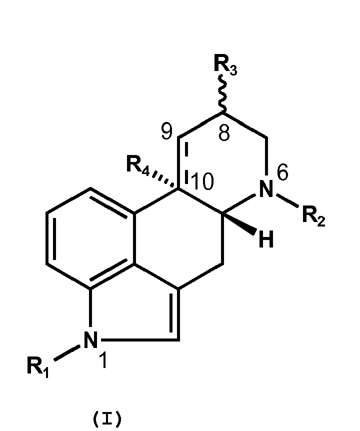 wherein
R₁ is hydrogen, C₁₋₄-alkyl, C₂₋₄-alkenyl, C₂₋₄-alkinyl or C₁₋₄-acyl, and
R₂ is hydrogen, C₁₋₄-alkyl, C₂₋₄-alkenyl, C₂₋₄-alkinyl or C₁₋₄-acyl - if R₁ is allyl, then methyl, ethyl, n-propyl, i-propyl an allyl are excluded for R₂- and
R₃ is either
   (a) NH-C(X)-NR₅R₆, wherein X is S or O and R₅ and R₆ are each independently hydrogen or a C₁₋₄-alkyl, or R₅ and R₆ together with the connecting N-atom form a 5- to 10-membered heterocyclic ring, preferably a 5- or 6-membered ring, which optionally can contain further heteroatoms, the 8-substituent being in the α- or β-position, with the restriction that R₁ is always C₂₋₄-alkinyl; or
   (b) CO-NR₇-C(O)-NH-R₈, with R₇ and R₈ independently representing an alkyl group having from 1 to 4 carbon atoms or a (CH₂)ₙN(CH₃)₂ group wherein n is an integer from 1 to 3, and the 8-substituent being in the α- or β-position -with the exception of R₁ being hydrogen or methyl if R₂ is allyl-; or
   (c) CH₂O-R₉, with R₉ being C(O)-C₅H₄NBr and the 8-substituent being in the α- or β-position; and R₄ being hydrogen or OCH₃, and the bond --- between C atoms 9 and 10 is either a single or a double bond while the hydrogen or methoxy group at C10 (R₄) is omitted - with the exception of R₁ being hydrogen or methyl.

The term "alkyl" can be either a straight chain or branched hydrocarbon group.

Further preferred embodiments are described in the dependant claims. In other words, it is preferred to have ergot alkaloid derivatives according to the general structural formula (I), wherein the residue R₁ is C₂₋₄-alkyl, C₂₋₄-alkenyl or C₂₋₄-alkinyl. A preferred embodiment refers to R₁ being C₃-alkinyl (propinyl=propargyl), ethyl or allyl. Furthermore, the residue R₂ being methyl is preferred. Relating to the residue R₄, it is a preferred embodiment when the residue R₄ is hydrogen or methoxy or is omitted due to a double bond between C9 and C10. In another preferred embodiment with respect to the residues R₅ and R₆, these residues each independently are a methyl, ethyl, i-propyl, or s-butyl group.

Especially for R₃ = NH-C(X)-NR₅R₆, it is preferred when R₁ is propargyl (propinyl), wherein R₂, R₄, R₅, R₆ have the same meaning as mentioned above and X is oxygen or sulphur and the residue R₃ stands in 8α- or 8β-position. A very preferred embodiment relating to the compounds with R₃ = NH-C(X)-NR₅R₆ refers to R₁= propargyl (propinyl), R₂= methyl, R₄= hydrogen or double bond between C9 and C10 when R₄ is omitted, R₅, R₆= ethyl and X=oxygen, wherein R₃ is an residue as mentioned above standing in 8α-position.

Relating to the compounds with R₃ = CO-NR₇-C(O)-NH-R₈ it is preferred when R₁ represents the same residues as mentioned above, but without hydrogen and methyl and R₂, R₄, R₇ and R₈ have the same meaning as mentioned above, wherein R₃ stands in 8α- or 8β-position. Especially, it is preferred when R1 is ethyl or allyl, R₂ is methyl, R₃ has the same meaning as mentioned above and stands in 8β-position, R₄ is hydrogen or omitted in case of a double bond between C9 and C10, R₇ represents (CH₂)₃-N(CH₃)₂ and R₈ is ethyl.

For the compounds with R₃ = CH₂OR₉ and with R₉ being C(O)-C₅H₄NBr (i.e. bromonicotinic acid), it is preferred when R₁ has the same meaning as mentioned above, but without hydrogen and methyl and R₂, R₄ and R₉ have the same meaning as mentioned above and the substituent R₃ stand in 8α- or 8β-position. Especially, it is preferred for these compounds when R₁ is ethyl or allyl, R₂ is methyl, R₄ is hydrogen, methoxy or R₄ is omitted in case of a double bond between C9 and C10, R₉ has the same meaning as mentioned above. The residue R₃ stands in 8β-position.

Preferred compounds are namely 3-(1-propinyl-9,10-didehydro-6-methylergolinyl-8α-yl)-1,1-diethylurea;
3-(1-propinyl-6-methylergolinyl-8α-yl)-1,1-diethylurea;
3-(1-propinyl-9,10-didehydro-6-methylergolinyl-8β-yl)-1,1-diethylurea;
3-(1-propinyl-6-methylergolinyl-8β-yl)-1,1-diethylurea;
1-[(1-ethyl-6-methyl-ergolin-8β-yl)carbonyl]-1-[3-(dimethylamino)-propyl]-3-ethylurea;
1-[(1-allyl-6-methyl-ergolin-8β-yl)carbonyl]-1-[3-(dimethylamino)-propyl]-3-ethylurea;
1-[(1-ethyl-6-methyl-9,10-didehydro-ergolin-8β-yl)carbonyl]-1-[3-(dimethylamino)-propyl]-3-ethylurea;
1-[(1-allyl-6-methyl-9,10-didehydro-ergolin-8β-yl)carbonyl]-1-[3-(dimethylamino)-propyl]-3-ethylurea;
1-allyl-10α-methoxy-1,6-dimethylergoline-8β-methanol 5-bromonicotinate ester or
1-ethyl-10α-methoxy-1,6-dimethylergoline-8β-methanol 5-bromonicotinate ester.

It is a further object of the present invention to provide a method how to use effective compounds in a way that effective neuroprotection is ensured.

This problem was solved by the use of an ergot alkaloid derivative of the general formula (I) or a pharmaceutically acceptable salt thereof 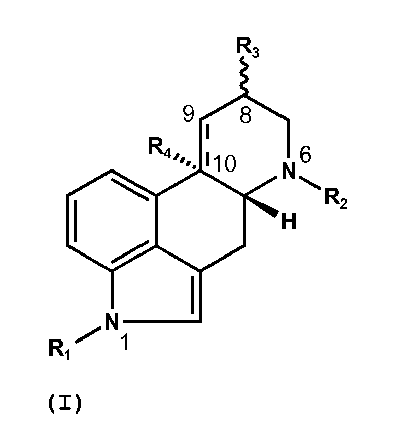 wherein
R₁ is hydrogen, C₁₋₄-alkyl, C₂₋₄-alkenyl, C₂₋₄-alkinyl or C₁₋₄-acyl, and
R₂ is hydrogen, C₁₋₄-alkyl, C₂₋₄-alkenyl, C₂₋₄-alkinyl or C₁₋₄-acyl and
R₃ is either
   (a) NH-C(X)-NR₅R₆, wherein X is S or O and R₅ and R₆ are each independently hydrogen or a C₁₋₄-alkyl, or R₅ and R₆ together with the connecting N-atom form a 5- to 10-membered heterocyclic ring, preferably a 5- or 6-membered ring, which optionally can contain further heteroatoms, the 8-substituent being in the α- or β-position; or
   (b) CO-NR₇-C(O)-NH-R₈, with R₇ and R₈ independently representing an alkyl group having from 1 to 4 carbon atoms or a (CH₂)ₙN(CH₃)₂ group wherein n is an integer from 1 to 3, the 8-substituent being in the α- or β-position; or
   (c) CH₂O-R₉, with R₉ being C(O)-C₅H₄NBr and the 8-substituent being in the α- or β-position; and R₄ being hydrogen or OCH₃, and the bond --- between C atoms 9 and 10 is either a single or a double bond while the hydrogen or methoxy group at C10 (R₄) is omitted, which is used
at a dosage that achieves a continuous infusion or release rate between 0,005 and 5,000 mg per day depending on the ergot alkaloid derivative resulting in a constant blood plasma concentration of 10 pg/ml to 10 ng/ml of the at least one ergot alkaloid derivative or its pharmaceutically acceptable salt, which is sustained each day for 24h. Treatable diseases are Parkinson's disease and other forms of chronic neuronal death and neurodegeneration such as Alzheimer's disease, Huntington's disease, Amyotrophic lateral sclerosis (ALS) or Multiple system atrophy (MSA).

In a preferred embodiment an ergot alkaloid derivative or a pharmaceutically acceptable salt thereof is used, wherein the at least one ergot alkaloid derivative is a compound of the general formula (I) 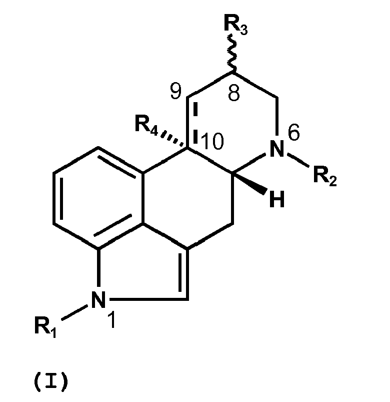 wherein
R₁ is hydrogen, C₁₋₄-alkyl, C₂₋₄-alkenyl, C₂₋₄-alkinyl or C₁₋₄-acyl and
R₂ is hydrogen, C₁₋₄-alkyl, C₂₋₄-alkenyl, C₂₋₄-alkinyl or C₁₋₄-acyl and
R₃ is either
   (a) NH-C(X)-NR₅R₆, wherein X is S or O and R₅ and R₆ are each independently hydrogen or a C₁₋₄-alkyl, or R₅ and R₆ together with the connecting N-atom form a 5- to 10-membered heterocyclic ring, preferably a 5- or 6-membered ring, which optionally can contain further heteroatoms, the 8-substituent being in the α- or β-position, -with the restriction that R1 is always C₂₋₄-alkinyl; or
   (b) CO-NR₇-C(O)-NH-R₈, with R₇ and R₈ independently representing an alkyl group having from 1 to 4 carbon atoms or a (CH₂)ₙN(CH₃)₂ group in which n is an integer from 1 to 3, the 8-substituent being in the α- or β-position, -with the exception of R₁ being hydrogen or methyl if R₂ is allyl-; or
   (c) CH₂O-R₉, with R₉ being C(O)-C₅H₄NBr and the 8-substituent being in the α- or β-position; and R₄ being hydrogen or OCH₃, and the bond --- between C atoms 9 and 10 is either a single or a double bond while the hydrogen or methoxy group at C10 (R₄) is omitted.

Relating to the above mentioned use, it is further preferred when the at least one ergot alkaloid derivative or its pharmaceutically acceptable salt have no R₂= methyl, ethyl, n-propyl, i-propyl and allyl residue when R₁ is allyl. In another preferred use-embodiment, the at least one ergot alkaloid derivative or its pharmaceutically acceptable salt is administered at a dosage that achieves a continuous infusion or release rate between 0,05 and 0,5 mg per day depending on the ergot alkaloid derivative, which results in a constant blood plasma concentration of 100 pg/ml to 1 ng/ml of the at least one ergot alkaloid derivative or its pharmaceutically acceptable salt, which is sustained each day for 24h. The method can be used for treatment of Restless Legs Syndrome. Another option is the use for the prevention and/or treatment of serotonin induced potentation of thrombocyte aggregation. A preferred use is also the treatment of mental diseases such as psychosis, depression and other behavioural and cognitive disorders.

A further preferred use is the treatment of neurodegenerative diseases such as Parkinson's disease, Alzheimer's disease, Huntington's disease, Amyotrophic lateral sclerosis (ALS) or Multiple system atrophy (MSA) as well as the use for the treatment of migraine.

Why would compounds, that apparently display some neuroprotective activity in neuronal cell culture, fail to show any apparent effect in PD patients, is difficult to explain. Among the numerous possibilities that could account for this lack of efficiency, the following two options appear the most plausible factors to us:
a) even the maximum concentration of the neuroprotective agent, assumed to act stoichiometrically at the targeted site in the brain, is too low to exert the desired effect
b) even if a sufficient concentration can be reached at the targeted neurons, the initial concentration of the neuroprotective agent, cannot be maintained for long enough to effectively counteract the deleterious action of free radicals in the affected brain structure throughout the entire day.

Hence, the therapeutic intervention by exogeneous agents should concentrate at the dysfunctional site in the brain to ensure that the compound (i.e. a scavenger of free radicals) becomes available at that site in a sufficient concentration and remains there for long enough to slow down the chronic progression of a neurodegenerative condition as exemplified by PD. Such counteractive strategy should function independently of what the initial cell death trigger (exogeneously or genetically-induced excitotoxicity, proteasome or mitochondrial disorders other mutations toxins etc.) actually was.

However, it will be much easier to implement effective countermeasures to slow down the progression of a neurodegenerative disease if the primary site of the neuronal death and the type of neurons that are the first to die have already been identified. This precondition is fulfilled in some neurodegenerative diseases including the early phase of the PD pathology, where the observable cell death predominates in monoaminergic neurons located in the substantia nigra and nucleus accumbens (dopamine), the locus coeruleus (noradrenaline) and the nucleus raphe dorsalis (5-HT).

The cell death selectivity that characterize the early stage of neurodegenerative disorders as manifested in PD offers a chance for developing compounds that can be accumulated by the affected neuronal type either by an active uptake mechanism and/or in the form of specific binding to various receptor types located at the outer membranes or neurotransmitter transport of these neurones. Compounds eligible for such a role should posses high affinity for dopaminergic receptors of the D1 and D2 variety because these receptor types are numerous in the basal ganglia and basal ganglia are primary targets of neurodegenerative processes in PD.
The second requirement would be their ability to function as free oxyradical scavengers by incorporating one or more oxygen atoms into their chemical structure. Hence, an ideal compound would be a small molecule, impervious to metabolic degradation, that can easily penetrate blood brain barrier and attaches itself to those neurones that are principally threatened by the neurodegenerative condition. Furthermore, these compounds should remain attached to the target structure in the brain for as long as they are capable of accepting oxyradicals. When fully oxydised, the affinity of these compounds for whatever protein structure should alter in such a way as to promote their dissociation from these structures. This dissociation should prevent already oxidised compounds from blocking the access to these binding sites by yet unaltered parent anti-oxidant molecules. The presence of the proposed compounds at the target neuronal structures should be sustained rather than transient because leaving neurones 'unprotected' for any length of time may not stop the progressive cell death. The reason for the stipulation is our observation that leaving the neurones unprotected for even couple of hours e.g. after oral application of drugs, can be expected to be detrimental to the ultimate survival of these cells. Furthermore, isomers or stereomers of these compounds can be also used as anti-oxidants as even these compounds. They may display different affinities for monoamine receptors. To avoid the apparent deficiency of achieving sustained levels of the active compounds at the receptors that results from oral intake, the mode of administration of the compound should favour a sustained action at the target neurones covering all 24 hours per day.

One additional bonus offer by these compounds can be derived from their ability to activate D3 receptors at extremely low concentrations. Such an action mobilises stem or progenitor cells e.g. in the subependymal layer in the brain to potentially participate in neuronal repair.
Finally, any neuroprotection offered by such a therapy needs to be provided for the rest of the life of the affected patients, i.e. for a very long time. Thus adverse effects, even minor, cannot be tolerated for the many years as they would jeopardize patients' compliance and thus not be useful for neuroprotection. Free radicals are known to play important and also helpful roles in the organism. One additional great advantage of our invention is the surprising finding that the compounds according to the present invention accumulate where they are needed leaving the rest of the body unaffected (so that e.g. macrophages still can effectively fight foreign in orders and infections but also degenerated or even malignant cells in all other parts of the body.

The invention approximates some of the requirements listed above. The compounds as described above are small molecules derived from natural ergot alkaloids of the 8-α-ergolines variety with low toxicity but high affinity for dopaminergic receptors and capable of functioning as oxygen radical scavengers (each molecule is capable of taking up four or even five oxygen atoms) Based on extensive studies, it was found that the compounds according to the present invention can function as potent scavengers of free radicals, for example, in human brain. They showed an outstanding affinity for dopaminergic and/or other monoaminergic receptors, which should facilitate therapeutic targeting of those brain structures that are affected by neurodegenerative processes participating, e.g., in the etiology and progression of Parkinson's disease and other neurodegenerative disorders. It was found that these compounds have the ability to selectively bind and inactivate free radicals immediately in the vicinity of those neurones (in particular dopaminergic) or within them that are subjected to oxidative stress and possibly to a metabolic overload. This ability is intended to promote the efficiency of eventual therapeutic application in the treatment of Parkinson's disease and other neurodegenerative diseases and for the prevention or attenuation of their progression. Each molecule of the compounds according to the present invention can, via an oxidative pathway, bind and neutralize up to six oxygen radicals and thus function as radical scavengers. Various ways to achieve a sustained protection of specific neurons against oxidative stress were investigated. It was found that only acting in effective concentration 24 hours a day can ensure effective neuroprotection, for example, in humans. This new observation clearly explains why many therapeutic attempts were doomed to fail. This was because their anti-oxidant or other effects could not be maintained for a sufficient length of time. The compounds of the general structural formula (I) may optionally be converted into pharmaceutically acceptable salts using the common procedures. The preferred formulation include sulphate, bisulphate, nitrate, phosphate, hydrogen phosphate, hydrochloride, hydrobromide, hydroiodide, acetate, tartrate, lactate, citrate, gluconate, fumarate, maleate, hydroxyl maleate, succinate, pamoate, benzoate, propionate, pyruvate, oxalate, malonate, cinnamate, salicylate, alkyl sulphonate, aryl sulphonate, and aralkyl sulphonate.

The substances according to the present invention with their high affinity for the various subtypes of dopamine receptors (D1, D2 and D3) are not only suitable for treating Parkinson's disease but also any other neurodegenerative conditions as described and wherever ROS scavenging properties may interrupt pathogenetic cascades. Other treatable diseases are already mentioned above. It is very helpful that apart from lisuride and terguride, some of the other structures described here also induce potent symptomatic effects, which may actually improve patients' compliance. Furthermore, the proposed compounds may also alleviate sleep disorders such as Restless Legs Syndrome (RLS), lower prolactin and act as migraine-prophylactic. These beneficial CNS effects may greatly improve patients compliance during a long-term therapy.

The pharmaceutical compounds of this invention or its pharmaceutically compatible acid addition salts according to the present invention are intended for oral, sublingual, transdermal, rectal, topical, and parenteral (such as intravenous) application that is able to ensure a full protection. This includes all forms of continuous dopaminergic stimulation.

The pharmaceutical compounds of this invention can be administered also in a special depot form facilitating controlled and continuous release of the active ingredient, such as a offered by a transdermal patch, implants or parenteral or intra-enteral infusion. Any of these or other application forms is is to be preferred because it guarantees a sustained exposure of vulnerable cells to these anti-oxidants.

Dosage of the pharmaceutical compounds of this invention should conform to the needs of the treated patient, the severity of his symptoms and the form of administration. The effective daily dose for the oral, sublingual, transdermal, rectal, topical, and parenteral administration should typically fall within the range of 0.001 to 20 mg per kg of body weight. Especially, a dosage should be used that achieves a continuous infusion or release rate between 0,005 and 5,000 mg per day depending on the ergot alkaloid derivative, which results in a constant blood plasma concentration of 10 pg/ml to 10 ng/ml of the at least one ergot alkaloid derivative or its pharmaceutically acceptable salt, which is sustained each day for 24h. Preferred is a dosage that achieves a continuous infusion or release rate between 0,05 and 0,5 mg per day depending on the ergot alkaloid derivative, which results in a constant blood plasma concentration of 100 pg/ml to 1 ng/ml of the at least one ergot alkaloid derivative or its pharmaceutically acceptable salt, which is sustained each day for 24h. According to the form of their intended application, the pharmaceutical compounds are to be produced in a standard way by utilising solid or liquid substrates and adjuvants common in pharmaceutical production.

Substrates and adjuvants may include binding agents, fillers, tabletting aids, diluents, solubility promoters, dyes, flavouring substances, wetting agents, emulgators, pH buffer additions, suspension aids, non-aqueous adjuvants and preservatives.

Cellulose, mannitol, and lactose are preferably used as fillers. Solubility promoters may include a starch or its derivatives or polyvinyl pyrrolidone. Adding EDTA to a solution of the active ingredient is beneficial. Sodium lauryl sulphate, lecithin, sorbitan monooleate, and gum arabic are all eligible as emulgators. A suspension aid may be selected from sorbitol, methyl cellulose, gelatin, hydroxyethyl cellulose, carboxymethyl cellulose, aluminium stearate gel and hydrated nutrient fats. Potential non-aqueous adjuvants may constitute any of the following substances: almond oil, coconut oil, glycerin ester, propylene glycol, or ethyl alcohol. Suitable preservatives are methyl-p-hydroxybenzoate, ethyl-p-hydroxybenzoate, sodium acid sulfite, and ascorbic acid. Magnesium stearate may be used as a lubricant.

The active ingredient oaccording to the present invention can be applied orally as solids administered in the form of tablets, capsules, agglomerations, powders, and lozenges. Other application in a liquid form may include aqueous solutions, suspensions, syrups, or soluble powders and oral sprays.

Parenteral applications of the active ingredient of the general structural formula (I) may include subcutaneous, intramuscular, or intravenous injections applied in the form of a liquid suspension or a solution. The active ingredients could be either dissolved or suspended shortly prior to injection. Addition of emulgators or wetting agents to a suspension is hoped to achieve a more even distribution of the active ingredient in a given liquid. The preferred concentration of the active ingredient in either saline solutions or glycerin is 0.1 to 10%.

The active ingredient according to the present invention, when used or transdermal application, is distributed in a carrier matrix of mineral or paraffin oil, a polyacrylate, or a wax. The matrix may also contain a transdermal transport enhancer and/or structure breaker such as dimethyl sulfoxide or propylene glycol.

## Claims

1. Ergot alkaloid derivatives of the general formula (I) or pharmaceutically acceptable salts thereof 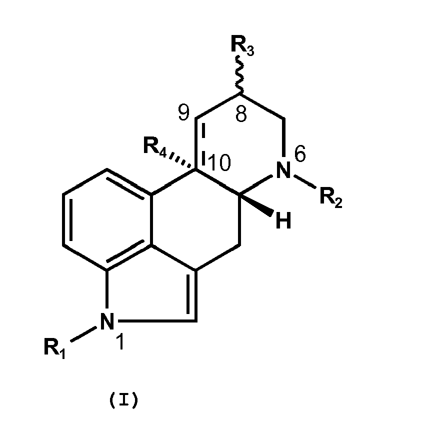 wherein
R₁ is C₂₋₄-alkyl, C₂₋₄-alkenyl or C₂₋₄-alkinyl, and
R₂ is hydrogen, C₁₋₄-alkyl, C₂₋₄-alkenyl, C₂₋₄-alkinyl or C₁₋₄-acyl - if R₁ is allyl, then methyl, ethyl, n-propyl, i-propyl and allyl are excluded for R₂- and R₃ is
CO-NR₇-C(O)-NH-R₈, with R₇ and R₈ independently representing an alkyl group having from 1 to 4 carbon atoms or a (CH₂)ₙN(CH₃)₂ group wherein n is an integer from 1 to 3,
and the 8-substituent being in the α- or β-position; and
R₄ being hydrogen or OCH₃, and
the bond --- between C atoms 9 and 10 is either a single or a double bond while the hydrogen or methoxy group at C10 (R₄) is omitted.

2. Ergot alkaloid derivatives according to claim 1 wherein the residue R₁ is C₃-alkinyl (propinyl=propargyl), ethyl or allyl.

3. Ergot alkaloid derivatives according to claim 1 or 2 wherein the residue R₂ is methyl.

4. Ergot alkaloid derivatives according to one of the claims 1 to 3 wherein the residue R₄ is hydrogen or methoxy or wherein the residue R₄ is omitted due to a double bond between C9 and C10.

5. Ergot alkaloid derivatives according to one of the claims 1 to 4 wherein the residues R₅ and R₆ each independently are a methyl, ethyl, i-propyl, or s-butyl group.

6. Ergot alkaloid derivatives according to one of the claims 1 to 5, namely
1-[(1-ethyl-6-methyl-ergolin-8β-yl)carbonyl]-1-[3-(dimethylamino)-propyl]-3-ethylurea;
1-[(1-allyl-6-methyl-ergolin-8β-yl)carbonyl]-1-[3-(dimethylamino)-propyl]-3-ethylurea;
1-[(1-ethyl-6-methyl-9,10-didehydro-ergolin-8β-yl)carbonyl]-1-[3-(dimethylamino)-propyl]-3-ethylurea or 1-[(1-allyl-6-methyl-9,10-didehydro-ergolin-8β-yl)carbonyl]-1-[3-(dimethylamino)-propyl]-3-ethylurea.

7. Use of an ergot alkaloid derivative of the general formula (I) or a pharmaceutically acceptable salt thereof 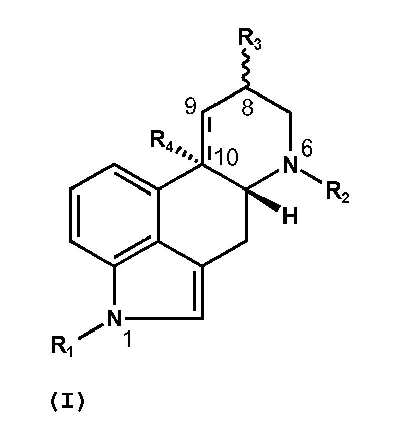 wherein
R₁ is C₂₋₄-alkyl, C₂₋₄-alkenyl or C₂₋₄-alkinyl, and
R₂ is hydrogen, C₁₋₄-alkyl, C₂₋₄-alkenyl, C₂₋₄-alkinyl or C₁₋₄-acyl -if R₁ is allyl, then methyl, ethyl, n-propyl, i-propyl and allyl are excluded for R₂- and
R₃ is CO-NR₇-C(O)-NH-R₈, with R₇ and R₈ independently representing an alkyl group having from 1 to 4 carbon atoms or a (CH₂)ₙN(CH₃)₂ group wherein n is an integer from 1 to 3,
the 8-substituent being in the α- or β-position;
and
R₄ being hydrogen or OCH₃, and
the bond --- between C atoms 9 and 10 is either a single or a double bond while the hydrogen or methoxy group at C10 (R₄) is omitted
for the treatment of Parkinson's disease and other forms of chronic neuronal death and neurodegeneration such as Alzheimer's disease, Huntington's disease, Amyotrophic lateral sclerosis (ALS) or Multiple system atrophy (MSA)
at a dosage that achieves a continuous infusion or release rate between 0,005 and 5,000 mg per day depending on the ergot alkaloid derivative resulting in a constant blood plasma concentration of 10 pg/ml to 10 ng/ml of the at least one ergot alkaloid derivative or its pharmaceutically acceptable salt, which is sustained each day for 24h.

8. Use of at least one ergot alkaloid derivative or its pharmaceutically acceptable salt according to claim 7, at a dosage that achieves a continuous infusion or release rate between 0,05 and 0,5 mg per day depending on the ergot alkaloid derivative resulting in a constant blood plasma concentration of 100 pg/ml to 1 ng/ml of the at least one ergot alkaloid derivative or its pharmaceutically acceptable salt, which is sustained each day for 24h.

9. Use of at least one ergot alkaloid derivative or its pharmaceutically acceptable salt according to claim 7 or 8 for treatment of Restless Legs Syndrome.

10. Use of at least one ergot alkaloid derivative or its pharmaceutically acceptable salt according to claim 7 or 8 for the prevention and/or treatment of serotonin induced potentation of thrombocyte aggregation.

11. Use of at least one ergot alkaloid derivative or its pharmaceutically acceptable salt according to claim 7 or 8 for the treatment of mental diseases such as psychosis, depression and other behavioural and cognitive disorders.

12. Use of at least one ergot alkaloid derivative or its pharmaceutically acceptable salt according to claim 7 or 8 for treatment of neurodegenerative diseases such as Parkinson's disease, Alzheimer's disease, Huntington's disease, Amyotrophic lateral sclerosis (ALS) or Multiple system atrophy (MSA).

13. Use of at least one ergot alkaloid derivative or its pharmaceutically acceptable salt according to claim 7 or 8 for treatment of migraine.
